# EUROPÄISCHE PATENTSCHRIFT

(11) **EP 2 645 968 B1**
(45) Veröffentlichungstag und Bekanntmachung des Hinweises auf die Patenterteilung: **12.03.2014**
(21) Anmeldenummer: 11790877.2
(22) Anmeldetag: 29.11.2011
(51) Int. Cl.: A61F 5/01

(54) **VERSTELLEINRICHTUNG FÜR EINE GELENKEINRICHTUNG**
ADJUSTING DEVICE FOR A JOINT MECHANISM
DISPOSITIF DE RÉGLAGE POUR UN DISPOSITIF ARTICULÉ

(30) Priorität: 03.12.2010 DE 102010053176
(43) Veröffentlichungstag der Anmeldung: 09.10.2013
(73) Patentinhaber: Otto Bock HealthCare GmbH, 37115 Duderstadt (DE)
(72) Erfinder: SCHILLING, Matthias, 37345 Weißenborn-Lüderode (DE)
(74) Vertreter: Stornebel, Kai
(86) Internationale Anmeldenummer: PCT/EP2011/005974
(87) Internationale Veröffentlichungsnummer: WO 2012/072231

(56) Entgegenhaltungen:
- DE-A1-102008 027 639
- US-B2- 7 534 220

## Beschreibung

Die Erfindung betrifft eine Verstelleinrichtung mit einem ersten Element und einem verlagerbar dazu gelagerten zweiten Element, das erste Element weist zumindest eine Rasteinrichtung auf, an dem zweiten Element ist zumindest ein mit einem Betätigungselement gekoppeltes Verriegelungselement verlagerbar angeordnet, das aus einer Freigabestellung außer Eingriff mit der Rasteinrichtung in eine Verriegelungsstellung in Eingriff mit der Rasteinrichtung bringbar ist. Eine solche Verstelleinrichtung kann als Gelenkeinrichtung insbesondere bei Orthesen oder Prothesen mit verschwenkbaren Gelenkelementen sinnvoll eingesetzt werden, insbesondere kann eine solche Gelenkeinrichtung an einer Orthese befestigt werden, um die Beweglichkeit des Gelenkes gezielt festzulegen.

Die US 5,358,469 beschreibt eine Orthese mit einer Gelenkeinrichtung, an der eine Schiene an einem ersten Gelenkelement und eine Schiene an einem zweiten Gelenkelement angeordnet sind. Über die Schienen erfolgt eine Festlegung an einer Gliedmaße, insbesondere an einem Bein. Die Gelenkeinrichtung weist eine gemeinsame Drehachse auf, um die die beiden Gelenkelemente relativ zueinander verschwenken. Der Gelenkeinrichtung ist eine Feder zugeordnet, die eine Vorspannung in Flexions- und/oder Extensionsrichtung bewirken kann, um eine Bewegung zu unterstützen oder ihr entgegenzuwirken. Eine Bewegungseinschränkung der Verschwenkung erfolgt über einen stiftförmigen Anschlag mit einem Gehäuse, in dem federbelastete Verriegelungsstifte angeordnet sind. In einem Gehäuse sind Löcher vorgesehen, in die die Verriegelungsstifte eingreifen, um den Umfang der Verschwenkbewegung zu begrenzen. Um die Anschlagposition zu verstellen, werden die Rückhaltestifte in das Gehäuse hineingedrückt und zu der gewünschten Bohrung verlagert.

Die US 7,534,220 B2 beschreibt eine Orthese mit zwei drehbar aneinander gelagerten Schienen. An einer Gelenkeinrichtung sind eine Unterschenkelschiene und eine Oberschenkelschiene angeordnet. Jede Schiene ist vorzugsweise über Nieten an der Gelenkeinrichtung befestigt. An jeder Schiene kann eine Verlängerung befestigt sein, die aus einem steifen Material, beispielsweise Stahl, hergestellt ist und eine Reihe aneinander beabstandeter Einstelllöcher aufweist. Sobald die gewünschte Längeneinstellung vorgenommen wurde, wird diese über einen Einschraubknopf oder einen Verriegelungshe-Verriegelungshebel fixiert. Um das Ausrichten der Einstelllöcher zu den Verriegelungselementen zu erleichtern, sind Erhebungen vorgesehen, die haptisch die korrekte Ausrichtung der Schienen zueinander anzeigen.

Die DE 10 2008 027 639 A1 betrifft ein Orthesengelenk zum Unterstützen eines anatomischen Gelenks mit einem Gelenkoberteil und einem gelenkig damit verbundenen Gelenkunterteil. Ein Sperrelement zum automatischen Entriegeln und Verriegeln des Orthesengelenkes in einer beliebigen Position sowie ein Betätigungselement für das Sperrelement sind vorgesehen. Ein Sensormittel zum Erfassen von für die Entriegelung und Verriegelung relevanter Informationen sind dem Betätigungselement zugeordnet. Das Sensormittel umfasst zumindest zwei Sensoren aus der Gruppe Neigungssensor, Drehwinkelsensor, Beschleunigungssensor und Gyroskop zum Erfassen von Bewegungs- und/oder Ruhezuständen eines Orthesengelenknutzers.

Der Aufbau einer solchen Gelenkeinrichtung ist aufwendig, die Verstellung der Anschlagposition erfordert feinmotorische Fähigkeiten und die Konstruktion ist anfällig für Verschmutzungen und Wassereintritt.

Aufgabe der vorliegenden Erfindung ist es, eine Verstelleinrichtung bereitzustellen, die einfach aufgebaut ist, zuverlässig funktioniert und eine schnelle Verstellung einer Anschlagsposition oder eines Lagerbolzens ermöglicht.

Erfindungsgemäß wird diese Aufgabe durch eine Verstelleinrichtung mit den Merkmalen des Hauptanspruches gelöst, vorteilhafte Ausgestaltungen und Weiterbildungen der Erfindung sind in den Unteransprüchen, der Beschreibung und den Figuren gezeigt.

Die erfindungsgemäße Verstelleinrichtung mit einem ersten Element und einem verlagerbar, z.B. schwenkbar oder verschieblich dazu gelagerten zweiten Element, wobei das erste Element zumindest eine Rasteinrichtung aufweist und an dem zweiten Element zumindest ein mit einem Betätigungselement gekoppeltes Verriegelungselement verlagerbar angeordnet ist, das aus einer Freigabestellung außer Eingriff mit der Rasteinrichtung in eine Verriegelungsstellung in Eingriff mit der Rasteinrichtung bringbar ist, sieht vor, dass das Betätigungselement zumindest einen Betätigungsmagneten aufweist, der einem mit dem Verriegelungselement gekoppelten Verriegelungsmagneten zugeordnet ist und das Verriegelungselement in die Freigabestellung und/oder Verriegelungsstellung und/oder aus der Freigabestellung und/oder Verriegelungsstellung bewegt. Durch die Ausgestaltung des Betätigungselementes mit einem Betätigungsmagneten ist es möglich, aktiv eine Verriegelung oder Entriegelung einer Verstelleinrichtung oder eine Verstellung eines Verriegelungselementes zu bewirken. Eine mechanische Belastung auf das Verriegelungselement wird durch den Magneten nicht ausgeübt, so dass ein mechanischer Verschleiß, wie bei der Anwendung von Zug- und Druckfedern, nicht erfolgen kann. Mit dem Betätigungsmagneten ist eine schnelle Umschaltung zwischen einer Freigabestellung und einer Verriegelungsstellung möglich, darüber hinaus muss keine unmittelbare mechanische Wirkverbindung zwischen dem Betätigungselement und dem Verriegelungselement vorliegen, so dass beispielsweise auch eine Anwendung in verkapselten Baugruppen möglich ist. Das Betätigungselement mit dem Betätigungsmagneten kann dabei das Verriegelungselement über den Verriegelungsmagneten aktiv in die Verriegelungsstellung hinein und wieder hinaus bewegen. Ebenfalls kann eine aktive Einnahme der Freigabestellung bzw. ein Entfernen aus der Freigabestellung erfolgen. Grundsätzlich ist es auch möglich, dass eine Vorzugsstellung des Verriegelungselementes vorhanden ist, beispielsweise durch die Schwerkraft oder eine Vorbelastung durch ein Elastomerelement, so dass vorzugsweise eine Verriegelungsstellung eingenommen wird. Dann bedient das Betätigungselement das Verriegelungselement nur in Richtung der Freigabestellung und ermöglicht dann ein bewusstes Verstellen der Position des Verriegelungselementes. Nach Wegfall einer magnetischen Wechselwirkung rastet das Verriegelungselement dann selbsttätig in die Rasteinrichtung ein. Auch ist eine umgekehrte Wirkungsweise möglich, so dass als Vorzugsposition die Freigabestellung vorliegt, während durch das Betätigungselement aktiv eine Verriegelungsstellung eingenommen wird. Das Verriegelungselement kann die Elemente zueinander festlegen. Es besteht alternativ oder ergänzend die Möglichkeit, dass das Verriegelungselement als ein Widerlager ausgebildet ist, an dem Komponenten gelagert sein können und dessen Position verstellbar ist.

Das Betätigungselement ist vorzugsweise verschiebbar oder verschwenkbar an der Verstelleinrichtung, insbesondere an dem zweiten Element, gelagert. Insbesondere kann das Betätigungselement an der Oberseite der Verstelleinrichtung an dem zweiten Element angeordnet sein, so dass es leicht zu erreichen ist und zudem ausreichend Raum für eine Betätigung vorhanden ist.

Das Verriegelungselement kann als ein verschieblich gelagerter Stift und die Rasteinrichtung als eine Ausnehmung ausgebildet sein, in die das Verriegelungselement eingreift, wenn es in die Verriegelungsstellung verlagert wurde. Auch ist es möglich, dass das Verriegelungselement korrespondierend zu einer alternativ geformten Rasteinrichtung ausgebildet ist und dadurch eine formschlüssige Verriegelung der beiden Elemente zueinander erreicht werden kann. Das Verriegelungselement kann beispielsweise als eine Klammer oder Spange ausgebildet sein, während die Rasteinrichtung als ein Vorsprung oder eine Verzahnung ausgebildet ist, in die das Verriegelungselement formschlüssig eingreift.

Der Betätigungsmagnet kann magnetisch umpolbar sein, wenn dieser als Elektromagnet ausgebildet ist, alternativ dazu kann der Betätigungsmagnet magnetisch umpolbar gelagert sein, insbesondere drehbar gelagert sein, so dass auf den Verriegelungsmagneten unterschiedliche Pole einwirken und je nach Position einmal eine anziehende und einmal eine abstoßende Wirkung realisiert wird. Häufig wird bei einer anziehenden Wirkung die Freigabestellung bewirkt, bei einer abstoßenden die Verlagerung in eine Verriegelungsstellung.

An dem Betätigungselement kann zumindest ein Haltemagnet angeordnet sein, der das Betätigungselement in der jeweiligen Stellung an dem zweiten Element hält und nicht primär die Aufgabe hat, den Verriegelungsmagneten anzuziehen bzw. abzustoßen. Es besteht grundsätzlich die Möglichkeit, dass mehrere Magnete in dem Betätigungselement angeordnet sind, beispielsweise zwei oder drei Magnete, von denen ein Magnet als Betätigungsmagnet und der andere Magnet oder die anderen Magnete als Haltemagnete dienen. Der Haltemagnet verhindert, dass ein unbeabsichtigtes Verlagern des Betätigungselementes aus der einmal eingestellten Position erfolgt.

Das Verriegelungselement kann zwischen zwei gegenpolig ausgerichteten Haltemagneten an dem zweiten Element angeordnet sein, wobei ein weiterer Haltemagnet mit gleicher Magnetpolung neben dem Haltemagnet mit einer von dem Verriegelungsmagnet abweichenden Magnetpolung angeordnet ist. Bei einer solchen Anordnung mit vier Magneten in einer Reihe ist es möglich, dass stets zwei an dem zweiten Element angeordnete Haltemagnete wirksam sind und darüber hinaus eine Verlagerung des Verriegelungselementes in die gewünschte Stellung ermöglicht wird. Der Verriegelungsmagnet und ein weiterer Haltemagnet weisen dabei die gleiche Magnetpolung auf, zwei neben dem Verriegelungsmagnet angeordnete Haltemagnete weisen eine umgekehrte Magnetpolung auf. Über die paarweise Magnetpolung und eine Anordnung von Haltemagneten in dem Betätigungselement kann eine Aktuierung des Verriegelungselementes und gleichzeitig sichere Festlegung mit zwei Haltemagnetpaaren in beiden Stellungen erfolgen.

Es besteht ebenfalls die Möglichkeit, dass das zweite Element zumindest teilweise magnetisierbar ist, beispielsweise eine magnetisierbare Wandung aufweist, so dass zumindest im Bereich der Festlegung des Betätigungselementes an dem zweiten Element eine Anhaftung an einer magnetisierbaren Oberfläche möglich ist. Durch die magnetisierbare Oberfläche hindurch kann das Verriegelungselement durch eine Wechselwirkung des Verriegelungsmagneten mit den Betätigungsmagneten, die gleichzeitig auch Haltemagnete sind, erfolgen.

Das Verriegelungselement kann federbelastet in dem zweiten Element gelagert sein, so dass eine Vorzugsstellung beim Wegfall der magnetischen Kräfte vorhanden ist. Ebenfalls kann bei einer magnetisierbaren Ausgestaltung des Gehäuses die Federkraft eine zumindest teilweise Kompensation der Haltekraft durch den Verriegelungsmagneten bewirken.

Die beiden Elemente können als Gelenkelemente um eine gemeinsame Schwenkachse gelagert sein, wobei die Elemente in einer gemeinsamen Schwenkebene angeordnet sind und eine flache, scheibenartige oder hülsenartige Form aufweisen können. Die Elemente können zueinander in zumindest einer Verschwenkrichtung federvorgespannt gelagert sein, um das menschliche Gelenk bei einer Beugung oder Streckung zu unterstützen. Die Verstelleinrichtung selbst muss dabei nicht eine tragende Funktion aufweisen, vielmehr kann sie als ein modulares Anbauteil an einer Orthese oder Prothese ausgebildet sein, so dass eine Federvorspannung in Flexionsrichtung oder Extensionsrichtung und damit eine Unterstützung der jeweiligen Bewegung realisiert werden kann.

Neben einer vollständigen Verriegelung der Verstelleinrichtung und damit auch gegebenenfalls der Prothese oder Orthese ist es möglich, dass die Verriegelungseinrichtung die Bewegungsgrenzen der gegeneinander verlagerbaren Element, z.B. des Gelenkes festlegt, also dass in Extensionsrichtung und Flexionsrichtung, ausgehend von einer Ausgangsstellung, die jeweiligen maximalen Winkelstellungen festgelegt werden. Entsprechendes gilt für verschiebliche Lagerungen. Die Verriegelungseinrichtung verriegelt dann eine Bewegung über einen bestimmten Winkel oder eine bestimmte Strecke hinaus und nur lässt eine eingeschränkte Verschwenkung oder Verschiebung zwischen den Grenzen, die durch die Verriegelungseinrichtung bzw. mehreren Verriegelungseinrichtungen in der Verstelleinrichtung festgelegt werden, zu.

Es können zwei Betätigungsmagnete unterschiedlicher Polung in dem Betätigungselement angeordnet sein, um die unterschiedlichen Stellungen des Verriegelungselementes zu bewirken, also eine Verlagerung aus der Freigabestellung in die Verriegelungsstellung und umgekehrt aus der Verriegelungsstellung in die Freigabestellung.

Nachfolgend werden Ausführungsbeispiele der Erfindung anhand der beigefügten Figuren näher erläutert. Gleiche Bezugszeichen bezeichnen gleiche Bauelemente. Es zeigen:
- Fig. 1 -: eine schematische, teilgeschnittene Draufsicht auf eine Verstelleinrichtung;
- Fig. 1a -: eine Seitenansicht gemäß Figur 1;
- Fig. 2 -: eine Schnittdarstellung eines Elementes im verriegelten Zustand;
- Fig. 3 -: eine Schnittdarstellung gemäß Figur 2 im entriegelten Zustand;
- Fig. 4 -: eine Variante der Erfindung im verriegelten Zustand;
- Fig. 5 -: ein Element gemäß Figur 4 im entriegelten Zustand;
- Fig. 6 -: eine schematische Seitenansicht eines Oberteils;
- Fig. 7 -: eine Draufsicht auf ein Oberteil;
- Fig. 8 -: eine Variante der Fig. 4;
- Fig. 9 -: eine Variante der Fig. 5;
- Fig. 10 -: eine Schnittansicht einer verschiebbaren Variante; sowie
- Fig. 11 -: eine Draufsicht gemäß Fig. 10.

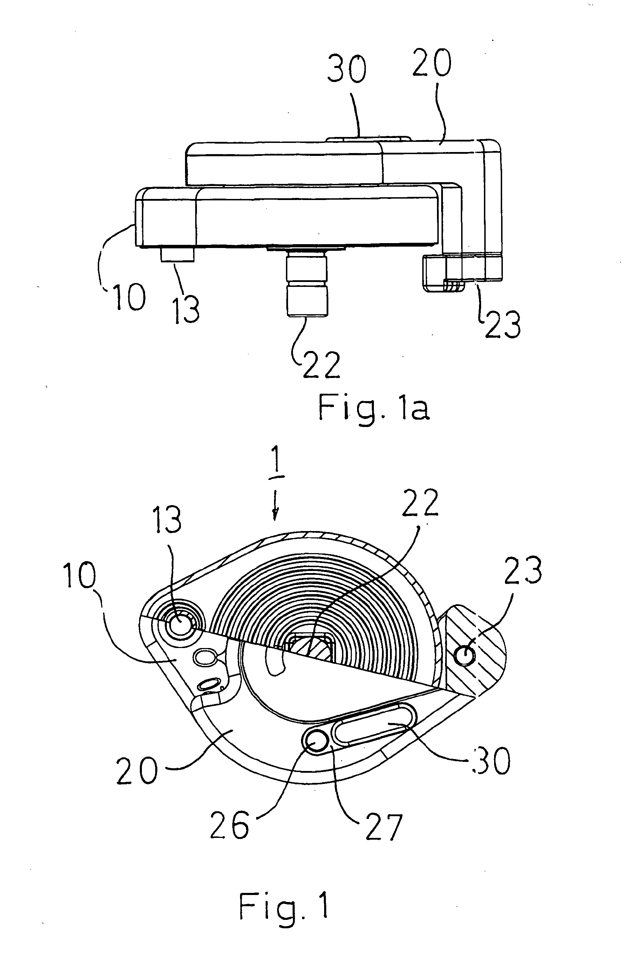

In der Figur 1 ist einer perspektivischen Darstellung eine Verstelleinrichtung 1 mit einem ersten Element 10 und einem zweiten Element 20 dargestellt. An dem ersten Element 10 ist eine Bohrung 13 ausgebildet, über die eine Festlegung beispielsweise an einer ersten Orthesenschiene erfolgen kann. Das zweite Element 20 ist um eine Schwenkachse 22 verschwenkbar an dem ersten Element 10 gelagert. Eine ebenfalls vorhandene Aufnahmebohrung 23 zur Festlegung an einer zweiten Orthesenschiene, die über ein separates Element mit der ersten Schiene verbunden sein kann, ist an dem zweiten Element 20 vorgesehen. Die Schwenkachse 22 verläuft vorzugsweise durch die Schwenkachse der Verstelleinrichtung, mit der die beiden Orthesenschienen verschwenkbar miteinander verbunden sind.

Das zweite Element 20 weist an seiner Oberseite ein Betätigungselement 30 auf, die verschiebbar in einer Führung 27 gelagert ist. Innerhalb der Führung ist ein Haltemagnet 26 zu erkennen, über den das Betätigungselement 30 in der eingestellten Position gehalten wird, wenn das Betätigungselement 30 in der Führung 27 nach links verschoben wird. Die Funktionsweise des Haltemagnets 26 sowie der Verstelleinrichtung wird weiter unten näher erläutert werden.

In der Figur 1a ist in einer Seitenansicht eine Verstelleinrichtung 1 gemäß Figur 1 dargestellt. Das erste Element 10 mit der Bohrung 13 ist unterhalb des zweiten Elementes 20 mit der Bohrung 23 angeordnet. An der Oberseite ist das Betätigungselement 30 zu erkennen. An der Unterseite, unterhalb des ersten Elementes, ragt die Schwenkachse 22 in Gestalt eines Zapfens vor. An dem Zapfen 22 können die Schienen oder Komponenten festgelegt werden. Der zweite Befestigungspunkt für beispielsweise die Orthesenschienen ist dann jeweils die Aufnahmebohrung 13, 23 des ersten oder zweiten Elementes 10, 20, so dass Momente übertragen werden können.

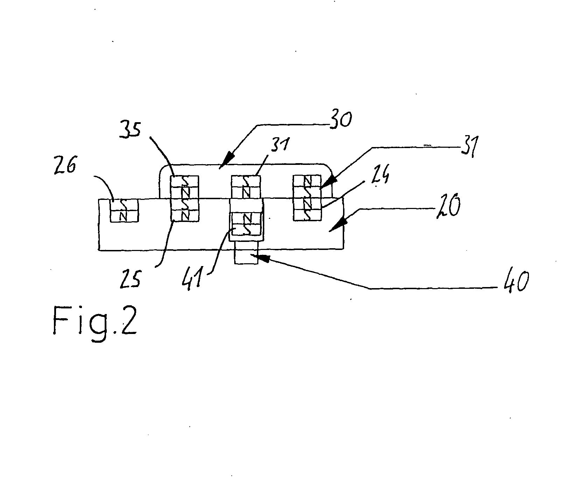

In der Figur 2 ist in einer schematischen Schnittdarstellung das zweite Element 20 in einer Teilansicht gezeigt. Üblicherweise liegt das zweite Element an der Außenseite der Verstelleinrichtung, so dass das Betätigungselement 30 auf der Außenseite der Verstelleinrichtung 1 liegt und damit leicht zugänglich ist. In der Ausführungsform gemäß Figur 2 ist das Betätigungselement 30 als ein Schiebeschalter ausgebildet, der an der Oberseite des zweiten Elementes 20 angeordnet ist. Innerhalb des Schiebeschalters 30 sind zwei Betätigungsmagnete 31 mit entgegengesetzter Magnetpolung angeordnet, der rechte Betätigungsmagnet 31 weist eine Nord/Süd-Polung auf, der mittlere Betätigungsmagnet 31 weist eine Süd/Nord-Polung auf, der linke Haltemagnet 35 weist ebenfalls eine Süd/Nord-Polung auf.

In dem zweiten Element 2 sind drei Haltemagnete 24, 25, 26 angeordnet, deren Magnetpolung so ausgerichtet ist, dass sie eine anziehende Wirkung auf die beiden äußeren Magnete 31, 35 des Betätigungselementes 30 haben. Das heißt, dass die Magnetpolung der Haltemagnete 24, 25 gleichgerichtet zu der Magnetpolung des rechten Betätigungsmagnetes 31 und des linken Haltemagnetes 35 ist. Der rechte Betätigungsmagnet 31 und der Haltemagnet 35 sind korrespondierend zu den Haltemagneten 24, 25 positioniert. Korrespondierend zu dem mittleren Betätigungsmagnet 31 des Betätigungselementes 30 ist ein Verriegelungselement 40 in Gestalt eines verschieblich gelagerten Bolzens in dem zweiten Element 20 angeordnet. Das Verriegelungselement 40 ragt an der Unterseite des zweiten Elementes 20 hervor, so dass es in eine nicht dargestellte Rasteinrichtung in dem ersten Element 10 eingreifen kann. Um eine Kraftkomponente nach unten in Richtung auf das zweite Element 10 ausüben zu können, ist in dem Verriegelungselement 40 ein Verriegelungsmagnet 41 angeordnet, dessen Magnetpolung so gewählt ist, dass eine Abstoßbewegung von dem mittleren Betätigungsmagneten 31 ausgeübt wird. Im dargestellten Ausführungsbeispiel sind die beiden Nordpole einander zugewandt.

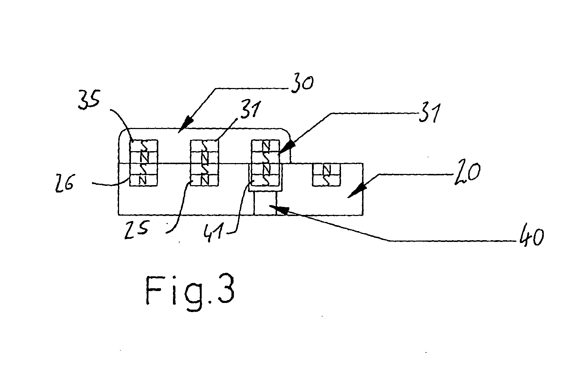

In der Figur 3 ist die Ausführungsform im entriegelten Zustand gezeigt, das Betätigungselement 30 ist nach links verschoben, so dass der linke Haltemagnet 35 auf dem außenliegenden Haltemagnet 26 zu liegen kommt. Aufgrund der gleichgerichteten Magnetpolung üben die beiden Haltemagnete 26, 35 eine Anziehungskraft aufeinander aus. Der mittlere Betätigungsmagnet 31 wird durch das Verschieben in die Entriegelungsstellung zur Deckung mit dem links neben dem Verriegelungselement 40 angeordneten Haltemagnet 25 gebracht und dort ebenfalls aufgrund der gleichgerichteten Magnetpolung sicher gehalten. Der rechte Betätigungsmagnet 31 wird durch das Verschieben in Deckung mit dem Verriegelungselement 40 und dem Verriegelungsmagneten 41 gebracht, auch hier erfolgt aufgrund der gleichgerichteten Magnetpolung der Magnete 31, 41 eine Anziehung der beiden Magnete und damit auch eine Verlagerung des Verriegelungselementes 40 in Richtung auf den Betätigungsmagnet 31. Dadurch ist es möglich, das Verriegelungselement 40 aus der Verriegelungsstellung gemäß Figur 2 in die Freigabestellung gemäß Figur 3 zu bewegen, so dass die beiden Elemente 10, 20 frei schwenkbar um die Schwenkachse 22 verdreht werden können.

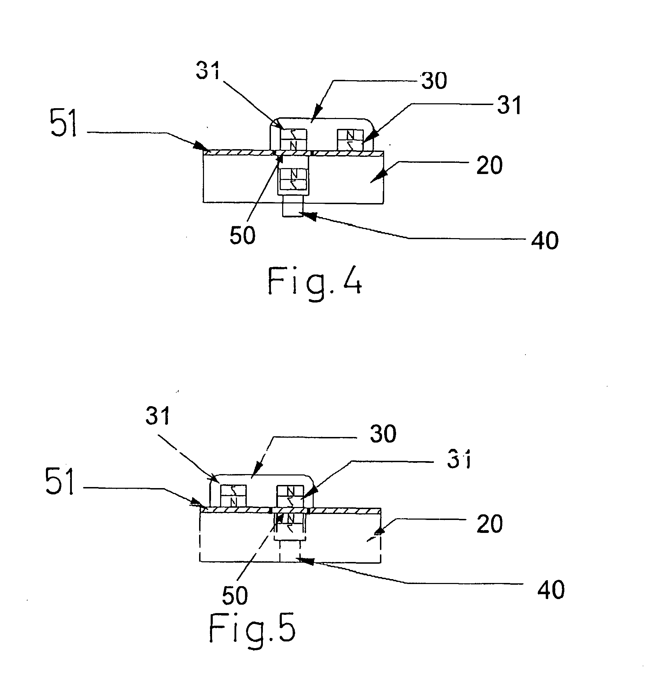

Eine Variante der Erfindung ist in den Figuren 4 und 5 dargestellt. Statt dreier Magnete in dem Betätigungselement 30 sind nur zwei Betätigungsmagnete 31 in dem Betätigungselement 30 angeordnet, die Magnetpolung der beiden Betätigungsmagnete 31 ist verschieden. An der Oberseite des zweiten Elementes 20 ist eine Beschichtung 51 aufgebracht oder eine Wandung 51 angeordnet, die magnetisierbar ist. Dadurch ist es möglich, dass das Betätigungselement 30 auf der Wandung 51 haftet. Die Wandung 51 kann sich über die gesamte Oberfläche des zweiten Elementes 20 erstrecken, alternativ ist nur eine bereichsweise Anordnung eines magnetisierbaren Materials an der Oberfläche des zweiten Elements 20 möglich. Im Bereich des Verriegelungsmagnetes 41 ist eine nicht magnetisierbare Schicht 50 angeordnet, die beispielsweise als Gleitschicht ausgebildet sein kann. Durch die Unterbrechung der magnetisierbaren Schicht 51 ist es möglich, dass beiderseits der neutralen Schicht 50 entgegengesetzte Polungen ausgebildet sein können, so dass das Betätigungselement 30 in jeder Stellung über zumindest ein Betätigungsmagnet 31 an dem zweiten Element 20 gehalten wird.

In der Figur 4 ist der Verriegelungszustand gezeigt, bei dem das Verriegelungselement 40 über die Unterseite des zweiten Elementes 20 hinausragt. Aufgrund der einander abstoßenden Magnetpolung des Betätigungsmagnetes 30 und des Verriegelungsmagnetes 41 wird das Verriegelungselement 40 in der Verriegelungsstellung gehalten.

In der Figur 5 ist das Betätigungselement 30 verschoben, der Betätigungsmagnet 31 mit der umgekehrten Magnetpolung ist zu dem Verriegelungselement 40 ausgerichtet und zieht über den Verriegelungsmagneten 41 das Verriegelungselement 40 nach oben. Die Wandung 50 dient als Anschlag.
Das Verriegelungselement 40 kann in Richtung auf eine Vorzugsstellung mit einer Federbelastung versehen sein, entweder in die Verriegelungsstellung oder in die Freigabestellung.

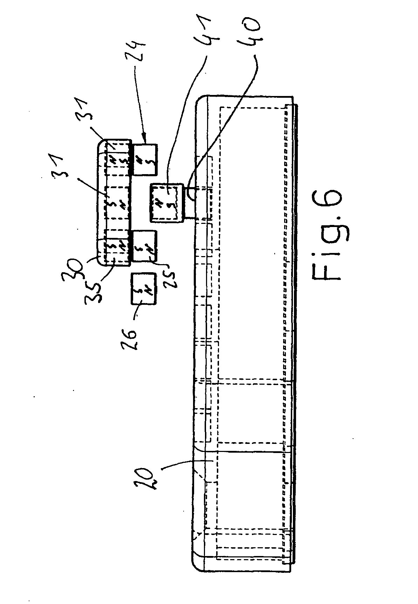

In der Figur 6 ist eine Seitenansicht eines zweiten Elementes 20 gezeigt. Das Betätigungselement 30 mit drei Magneten 31, 35 gemäß Figuren 2 und 3 ist ebenso zu erkennen wie die Anordnung und Orientierung des Verriegelungselementes 40 und des Verriegelungsmagneten 41 an der Oberseite des Verriegelungselementes 40.

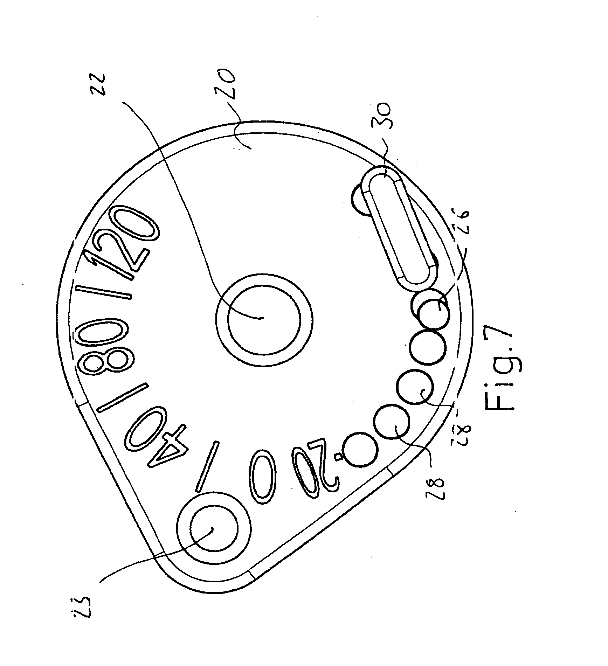

In der Figur 7 ist in einer Draufsicht das zweite Element 20 mit der Drehachse 22 und der Bohrung 23 für die Aufnahme beispielsweise einer Schraube zur Festlegung des zweiten Elementes 20 an einer Orthesenschiene zu erkennen. An der Oberseite sind das Betätigungselement 30 und ein Haltemagnet 26 zu erkennen. Das Betätigungselement 30 kann zusammen mit den Haltemagneten 24, 25, 26 und dem Verriegelungselement 40 mit dem Verriegelungsmagnet 41 in Ausnehmungen 28 an der Oberseite des Elementes 20 eingebracht werden, um unterschiedliche Anschlagpositionen des Verriegelungselementes 40 festzulegen. Dadurch ist es möglich, den Verschwenkwinkel des zweiten Elementes 20 relativ zu dem ersten Element 10 festzulegen.

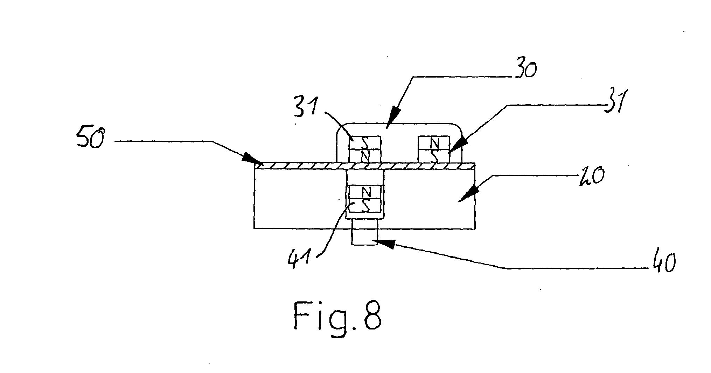

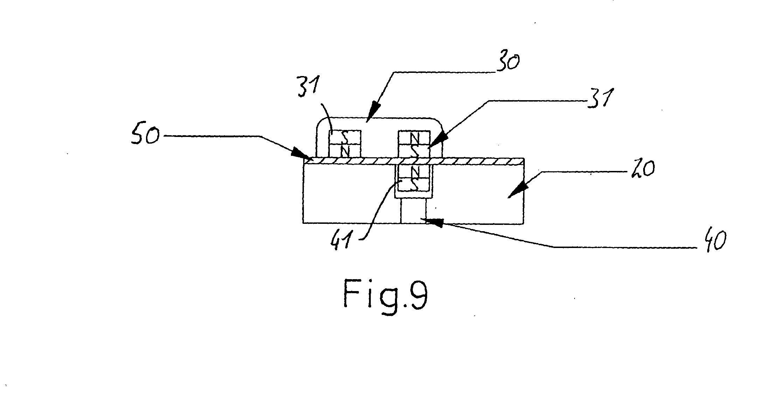

In den Figuren 8 und 9 ist eine Variante der Ausführungsform gemäß der Figuren 4 und 5 gezeigt. Statt einer magnetisierbaren Schicht 51, über die mittels der Betätigungsmagnete 31 das Betätigungselement 30 an dem zweiten Element 20 gehalten oder zusätzlich gesichert ist, ist in der Variante gemäß der Figuren 8 und 9 eine durchgängige Gleitschicht 50 angeordnet, die nicht magnetisierbar ist. Sofern das zweite Element 20 nicht aus einem magnetisierbaren Material besteht, wird das Betätigungselement 30 oder der Magnetschalter über eine nicht dargestellte Führung, beispielsweise eine Schwalbenschwanzführung, eine Nutführung oder dergleichen, an dem zweiten Element 20 gehalten.

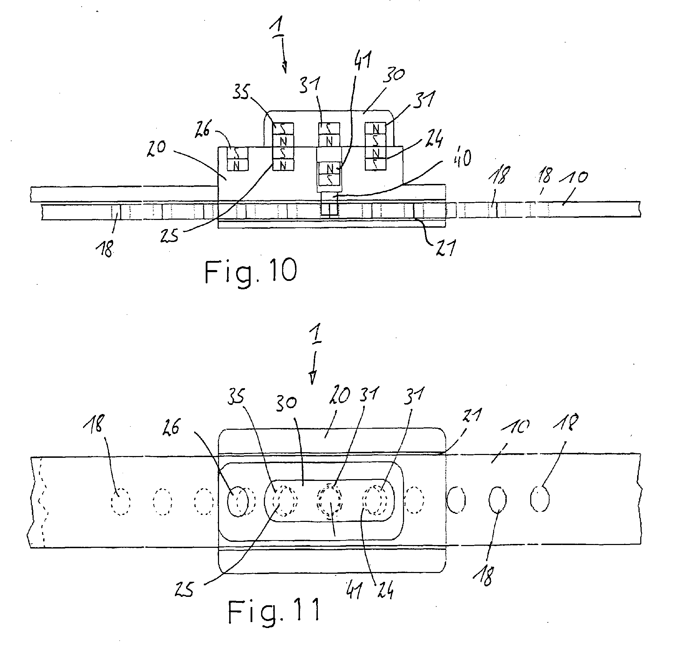

In der Figur 10 ist in einer Seitenansicht eine Variante der Verriegelungseinrichtung 1 gezeigt, bei der statt einer Lagerung um eine gemeinsame Schwenkachse die beiden Elemente 10, 20 verschieblich zueinander gelagert sind. Das erste Element 10 ist im Wesentlichen als eine flache Schiene mit Ausnehmungen 18 ausgebildet, in die das Verriegelungselement 41 eingreifen kann, wenn es sich in der Verriegelungsstellung befindet. In dem zweiten Element 20 ist eine Ausnehmung oder Führung 21 für die Aufnahme des ersten Elementes 10 ausgebildet. Innerhalb der Führung 21 kann das erste Element 10 entlang seiner Längserstreckung verschoben werden. In der Führung 21 können Beschichtungen zur Erleichterung der Relativbewegung zwischen dem ersten Element 10 und dem zweiten Element 20 vorgesehen sein.

Der Aufbau des zweiten Elementes entspricht im Wesentlichen dem der Figur 2, das Betätigungselement 30 in Gestalt eines Magnetschalters ist mit drei Magneten 31, 35 ausgestattet, von denen zwei als Betätigungsmagnete 31 und einer als Haltemagnet 35 ausgebildet sind. An der Oberfläche des zweiten Elementes 20, die der Unterseite des Magnetschalters 30 gegenüberliegt, sind drei Haltemagnete 24, 25, 26 angeordnet und darin festgelegt. Ein Verriegelungsmagnet 41 ist verlagerbar in einer Ausnehmung innerhalb des zweiten Elementes 20 angeordnet und wird in der Darstellung gemäß Figur 10 aufgrund der sich abstoßenden magnetischen Polung nach unten von dem Betätigungsmagnet 31 weggedrückt. Dadurch wird das Verriegelungselement 40 in Gestalt eines Zapfen in eine Ausnehmung 18 innerhalb des schienenartigen ersten Elementes 10 hineingedrückt und verriegelt das erste Element 10 formschlüssig gegen eine Verlagerungsbewegung relativ zu dem zweiten Element 20 und einer eventuell daran angeordneten Komponente.

In der Figur 11 ist die Anordnung gemäß Figur 10 in einer Draufsicht gezeigt.

Durch die Verstelleinrichtung 1 ist es somit möglich, nicht nur verdrehbar zueinander gelagerte Elemente 10, 20, sondern auch verschiebbare Elemente 10, 20 zu verstellen bzw. zu verriegeln. Über die Verstelleinrichtung 1 ist es möglich, durch eine einmalige Betätigung des Betätigungselementes 30 eine dauerhafte Verriegelung oder Entriegelung bereitzustellen, ohne dass durch den Nutzer weitere Kräfte aufgebracht werden müssten. Sobald sich das Betätigungselement 30 in der Entriegelungsstellung befindet, wird das Verriegelungselement 40 dauerhaft mit einer Kraft beaufschlagt, die das Verriegelungselement 40 aus einer formschlüssigen Verriegelung hinausbewegen möchte. Sobald eine Verlagerung des Verriegelungselementes 40 möglich ist, beispielsweise bei einer Entlastung einer Gelenkeinrichtung oder bei Nachlassen einer Zugkraft oder Druckkraft bei einer verschieblichen Lagerung zweier Elemente 10, 20 zueinander, wird die Verriegelung freigegeben und es kann eine Verstellung erfolgen. Umgekehrt drückt bei einer entsprechenden Stellung das Betätigungselement 30 der Betätigungsmagnet 31 das Verriegelungselement 40 in Richtung auf eine Ausnehmung 18, um eine formschlüssige Verriegelung zu bewirken. Sobald sich die Ausnehmung 30 in einer Stellung befindet, die zu der Bewegungsrichtung des Verriegelungselementes 40 korrespondiert, rastet das Verriegelungselement 40 in die Ausnehmung 18 ein und legt die beiden Elemente 10, 20 in der eingestellten Stellung zueinander fest.

## Patentansprüche

1. Verstelleinrichtung mit einem ersten Element (10) und einem verlagerbar dazu gelagerten zweiten Element (20), das erste Element (10) weist zumindest eine Rasteinrichtung (15) auf, an dem zweiten Element (20) ist zumindest ein mit einem Betätigungselement (30) gekoppeltes Verriegelungselement (40) verlagerbar angeordnet, das aus einer Freigabestellung außer Eingriff mit der Rasteinrichtung (15) in eine Verriegelungsstellung in Eingriff mit der Rasteinrichtung (15) bringbar ist, **dadurch gekennzeichnet, dass** das Betätigungselement (30) zumindest einen Betätigungsmagneten (31) aufweist, der einem mit dem Verriegelungselement (40) gekoppelten Verriegelungsmagneten (41) zugeordnet ist und das Verriegelungselement (40) in die Freigabestellung und/oder Verriegelungsstellung und/oder aus der Freigabestellung und/oder Verriegelungsstellung bewegt.

2. Verstelleinrichtung nach Anspruch 1, **dadurch gekennzeichnet, dass** das Betätigungselement (30) verschwenkbar oder verschiebbar an der Verstelleinrichtung (1) gelagert ist.

3. Verstelleinrichtung nach Anspruch 1 oder 2, **dadurch gekennzeichnet, dass** das Verriegelungselement (40) als verschieblich gelagerter Stift und die Rasteinrichtung (15) als Ausnehmung ausgebildet sind.

4. Verstelleinrichtung nach einem der voranstehenden Ansprüche, **dadurch gekennzeichnet, dass** der Betätigungsmagnet (31) magnetisch umpolbar oder magnetisch umpolbar gelagert ist.

5. Verstelleinrichtung nach einem der voranstehenden Ansprüche, **dadurch gekennzeichnet, dass** an dem Betätigungselement (30) zumindest ein Haltemagnet (35) angeordnet ist, der das Betätigungselement (30) in der jeweiligen Stellung an dem zweiten Element (20) hält.

6. Verstelleinrichtung nach Anspruch 5, **dadurch gekennzeichnet, dass** das Verriegelungselement (40) zwischen zwei gegenpolig ausgerichteten Haltemagneten (24, 25) an dem zweiten Element (20) angeordnet ist und ein weiterer Haltemagnet (26) mit gleicher Magnetpolung neben dem Haltemagnet (25) mit einer von dem Verriegelungsmagnet (31) abweichenden Magnetpolung angeordnet ist.

7. Verstelleinrichtung nach einem der voranstehenden Ansprüche, **dadurch gekennzeichnet, dass** das zweite Element (20) zumindest teilweise magnetisierbar ist.

8. Verstelleinrichtung nach einem der voranstehenden Ansprüche, **dadurch gekennzeichnet, dass** das Verriegelungselement (40) federbelastet in dem zweiten Element (20) gelagert ist.

9. Verstelleinrichtung nach einem der voranstehenden Ansprüche, **dadurch gekennzeichnet, dass** die Elemente (10, 20) um eine gemeinsame Schwenkachse und in zumindest einer Verschwenkrichtung federvorgespannt gelagert sind.

10. Verstelleinrichtung nach einem der voranstehenden Ansprüche, **dadurch gekennzeichnet, dass** die Verstelleinrichtung (1) als modulares Anbauteil einer Orthese oder Prothese ausgebildet ist.

11. Verstelleinrichtung nach einem der voranstehenden Ansprüche, **dadurch gekennzeichnet, dass** zumindest zwei Betätigungsmagnete (31) unterschiedlicher Magnetpolung in dem Betätigungselement (30) angeordnet sind.

## Claims

1. An adjusting device having a first element (10) and a second element (20), which is mounted so as to be displaceable with respect thereto, the first element (10) has at least one latching device (15), at least one locking element (40), which is coupled with an actuating element (30), is arranged so as to be displaceable on the second element (20), said locking element being movable out of a release position out of engagement with the latching device (15) into a locking position in engagement with the latching device (15), **characterized in that** the actuating element (30) has at least one actuating magnet (31) which is associated with a locking magnet (41) which is coupled with the locking element (40) and the locking element (40) moves into the release position and/or locking position and/or out of the release position and/or locking position.

2. The adjusting device as claimed in claim 1, **characterized in that** the actuating element (30) is mounted so as to be pivotable or displaceable on the adjusting device (1).

3. The adjusting device as claimed in claim 1 or 2, **characterized in that** the locking element (40) is realized as a slidably mounted pin and the latching device (15) is realized as a recess.

4. The adjusting device as claimed in one of the preceding claims, **characterized in that** the actuating magnet (31) can have its magnetic polarity reversed or is mounted so as to be able to have its magnetic polarity reversed.

5. The adjusting device as claimed in one of the preceding claims, **characterized in that** at least one holding magnet (35), which holds the actuating element (30) in the respective position on the second element (20), is arranged on the actuating element (30).

6. The adjusting device as claimed in claim 5, **characterized in that** the locking element (40) is arranged on the second element (20) between two holding magnets (24, 25) which are aligned with opposite poles and a further holding magnet (26) with the same magnetic polarity is arranged next to the holding magnet (25) with a magnetic polarity which deviates from the locking magnet (31).

7. The adjusting device as claimed in one of the preceding claims, **characterized in that** the second element (20) is magnetizable at least in part.

8. The adjusting device as claimed in one of the preceding claims, **characterized in that** the locking element (40) is mounted in a spring-loaded manner in the second element (20).

9. The adjusting device as claimed in one of the preceding claims, **characterized in that** the elements (10, 20) are mounted in a spring-preloaded manner about a common pivot axis and in at least one pivoting direction.

10. The adjusting device as claimed in one of the preceding claims, **characterized in that** the adjusting device (1) is realized as a modular attachment part of an orthesis or prosthesis.

11. The adjusting device as claimed in one of the preceding claims, **characterized in that** at least two actuating magnets (31) with different magnetic polarity are arranged in the actuating element (30).

## Revendications

1. Dispositif de réglage comprenant un premier élément (10) et un second élément (20) monté déplaçable par rapport à celui-ci, le premier élément (10) comportant au moins un moyen à enclenchement (15), avec au moins un élément de verrouillage (40) accouplé à un élément d'actionnement (30) et agencé déplaçable sur le second élément (20), élément de verrouillage qui est susceptible d'être amené depuis une position de libération hors d'engagement avec le moyen à enclenchement (15) jusque dans une position de verrouillage en engagement avec le moyen à enclenchement (15), **caractérisé en ce que** l'élément d'actionnement (30) comprend au moins un aimant d'actionnement (31) qui est associé à un aimant de verrouillage (41) accouplé à l'élément de verrouillage (40), et qui déplace l'élément de verrouillage (40) jusque dans la position de libération et/ou dans la position de verrouillage, et/ou hors de la position de libération et/ou de la position de verrouillage.

2. Dispositif de réglage selon la revendication 1, **caractérisé en ce que** l'élément d'actionnement (30) est monté avec possibilité de pivotement ou de translation sur le dispositif de réglage (1).

3. Dispositif de réglage selon la revendication 1 ou 2, **caractérisé en ce que** l'élément de verrouillage (40) est réalisé comme une tige montée en translation, et le dispositif à enclenchement (15) est réalisé comme un évidement.

4. Dispositif de réglage selon l'une des revendications précédentes, **caractérisé en ce que** l'aimant d'actionnement (31) est tel que ses pôles peuvent être magnétiquement inversés, ou est monté de telle façon que ses pôles peuvent être magnétiquement inversés.

5. Dispositif de réglage selon l'une des revendications précédentes, **caractérisé en ce qu'**au moins un aimant de maintien (35) est agencé sur l'élément d'actionnement (30), et maintient l'élément d'actionnement (30) à l'emplacement respectif sur le second élément (20).

6. Dispositif de réglage selon la revendication 5, **caractérisé en ce que** l'élément de verrouillage (40) est agencé sur le second élément (20) entre deux éléments de maintien (24, 25) orientés avec des pôles en sens contraire, et un autre aimant de maintien (26) avec des pôles magnétiques égaux est agencé à côté de l'aimant de maintien (25) avec des pôles magnétiques différents de l'aimant de verrouillage (31).

7. Dispositif de réglage selon l'une des revendications précédentes, **caractérisé en ce que** le second élément (20) est susceptible d'être au moins partiellement aimanté.

8. Dispositif de réglage selon l'une des revendications précédentes, **caractérisé en ce que** l'élément de verrouillage (40) est monté chargé par un ressort dans le second élément (20).

9. Dispositif de réglage selon l'une des revendications précédentes, **caractérisé en ce que** les éléments (10, 20) sont montés autour d'un axe de pivotement commun et précontraints par un ressort dans au moins une direction de pivotement.

10. Dispositif de réglage selon l'une des revendications précédentes, **caractérisé en ce que** le dispositif de réglage (1) est réalisé comme un composant rapporté modulaire d'une orthèse ou d'une prothèse.

11. Dispositif de réglage selon l'une des revendications précédentes, **caractérisé en ce qu'**au moins deux aimants d'actionnement (31) avec des pôles magnétiques différents sont agencés dans l'élément d'actionnement (30).
